# EUROPÄISCHE PATENTSCHRIFT

(11) **EP 3 790 839 B1**
(45) Veröffentlichungstag und Bekanntmachung des Hinweises auf die Patenterteilung: **10.04.2024**
(21) Anmeldenummer: 19721281.4
(22) Anmeldetag: 02.05.2019
(51) Int. Cl.: B81C 1/00

(54) **VERFAHREN ZUM HERSTELLEN EINER SEQUENZIEREINHEIT ZUM SEQUENZIEREN EINES BIOCHEMISCHEN MATERIALS**
METHOD FOR PRODUCING A SEQUENCING UNIT FOR SEQUENCING A BIOCHEMICAL MATERIAL
PROCÉDÉ DESTINÉ À FABRIQUER UNE UNITÉ DE SÉQUENÇAGE DESTINÉE À SÉQUENCER UN MATÉRIAU BIOCHIMIQUE

(30) Priorität: 08.05.2018 DE 102018207099
(43) Veröffentlichungstag der Anmeldung: 17.03.2021
(73) Patentinhaber: Robert Bosch GmbH, 70442 Stuttgart (DE)
(72) Erfinder: LAERMER, Franz, 71263 Weil Der Stadt (DE)
(86) Internationale Anmeldenummer: PCT/EP2019/061249
(87) Internationale Veröffentlichungsnummer: WO 2019/215006

(56) Entgegenhaltungen:
- GRÉGORY F. SCHNEIDER ET AL: "DNA Translocation through Graphene Nanopores", NANO LETTERS, Bd. 10, Nr. 8, 11. August 2010 (2010-08-11), Seiten 3163-3167, XP055156495, ISSN: 1530-6984, DOI: 10.1021/nl102069z
- DAISUKE MASHIYAMA ET AL: "Nanopatterning of Suspended Graphene Films by Local Catalytic Etching Using Atomic Force Microscopy Equipped with an Ag-Coated Probe", JOURNAL OF PHYSICAL CHEMISTRY C, Bd. 119, Nr. 21, 4. Mai 2015 (2015-05-04), Seiten 11914-11921, XP055549989, ISSN: 1932-7447, DOI: 10.1021/acs.jpcc.5b00884
- RAWLINGS COLIN ET AL: "High throughput lithography using thermal scanning probes", 2017 19TH INTERNATIONAL CONFERENCE ON SOLID-STATE SENSORS, ACTUATORS AND MICROSYSTEMS (TRANSDUCERS), IEEE, 18. Juni 2017 (2017-06-18), Seiten 418-422, XP033130786, DOI: 10.1109/TRANSDUCERS.2017.7994076 [gefunden am 2017-07-26]

## Beschreibung

### Stand der Technik

Die Erfindung geht aus von einem Verfahren nach Gattung des unabhängigen Anspruchs.

Bei sogenannten selbstorganisierende Monoschichten aus Präkursormolekülen, auch self-assembled monolayers oder kurz SAM genannt, docken die Präkursoren beispielsweise an Bindestellen auf einer Silizium- oder Glasoberfläche an, wobei abhängig von den Präkursoren auch eine Quervernetzung der Präkursormoleküle untereinander stattfinden kann. Diese Prozesse sind in der Regel selbstlimitierend, d. h., nach Abscheidung einer einzigen Monolage und Besetzung aller verfügbaren Bindungsplätze der Oberfläche findet keine weitere Abscheidung mit demselben Präkursor statt. Meistens wird dieses Verhalten dadurch erreicht, dass die Präkursormoleküle selektiv und ausschließlich auf hydrophilen Oberflächen andocken, dabei selbst eine hydrophobe Oberfläche erzeugen und damit die weitere Abscheidung selbsttätig stoppen, weil weitere Präkursormoleküle auf hydrophoben und damit wasserfreien Oberflächen nicht mehr andocken können.

Selbstorganisierende Monoschichten können auch aus der Flüssigphase mithilfe geeigneter Lösungsmittel, beispielsweise Hexan oder Heptan oder auch Fluorcarbonen wie etwa FC40, abgeschieden werden. Aufgrund der höheren Reinheit der Präkursor-Chemie und der besseren Qualität und Reproduzierbarkeit der abgeschiedenen Schichten hat sich jedoch die Gasphasenabscheidung durchgesetzt. Dabei werden flüssig vorgelagerte Präkursormoleküle in die Gasphase überführt, beispielsweise durch Erhitzen der Präkursorflüssigkeit. In einer Reaktionskammer wird der gewünschte Partialdruck des Präkursors eingestellt und mit dem Wafer in Kontakt gebracht. Es sind Methoden mit oder ohne Trägergas (Inertgas oder Edelgas wie beispielsweise N₂ oder Ar) möglich.

Gewisse SAM-Beschichtungen können auf Wafern in qualitativ hochwertige Graphenschichten umgewandelt werden. Dazu wird die SAM-Beschichtung nach der Abscheidung auf der Substratoberfläche beispielsweise durch Bedampfung mit einer 300 nm dicken Metallschicht, etwa aus Nickel oder Kupfer, überzogen und anschließend bei hoher Temperatur von 800 bis 1000 °C in Graphen umgewandelt.

Die Veröffentlichung DAISUKE MASHIYAMA ET AL: "Nanopatterning of Suspended Graphene Films by Local Catalytic Etching Using Atomic Force Microscopy Equipped with an Ag-Coated Probe",JOURNAL OF PHYSICAL CHEMISTRY C, Bd. 119, Nr. 21, 4. Mai 2015 (2015-05-04), Seiten 11914-11921,ISSN: 1932-7447, DOI: 10.1021/ acs.jpcc.5b00884 beschreibt ein Herstellungsverfahren zum Herstellen einer Sequenziereinheit zum Sequenzieren eines biochemischen Materials, wobei in einer Graphenschicht eine Sequenzierpore zum Sequenzieren des biochemischen Materials erstellt wird.

### Offenbarung der Erfindung

Vor diesem Hintergrund wird mit dem hier vorgestellten Ansatz ein Verfahren zum Herstellen einer Sequenziereinheit zum Sequenzieren eines biochemischen Materials gemäß dem Hauptanspruch vorgestellt. Durch die in den abhängigen Ansprüchen aufgeführten Maßnahmen sind vorteilhafte Weiterbildungen und Verbesserungen des im unabhängigen Anspruch angegebenen Verfahrens möglich.

Der hier vorgestellte Ansatz beruht auf der Erkenntnis, dass Nanoporen in einer Graphenschicht in einem thermischen Lithografieverfahren in einer geeigneten Vorläuferschicht, etwa einer selbstorganisierenden Monoschicht, vordefiniert und beim thermischen Umwandeln der Vorläuferschicht in die Graphenschicht je nach Umwandlungstemperatur auf einen passenden Durchmesser verkleinert werden können. Ein derart hergestelltes Element kann beispielsweise in eine Lab-on-Chip-Umgebung integriert werden, die quantitative Polymerasekettenreaktionen, kurz qPCR genannt, durchgeführt als LATEPCR (linear after the exponential), und die Sequenzierung von DNA-Fragmenten nach einer Sanger-Methode mit DNA-Fragmentlängenbestimmung über die Nanoporen kombiniert. Zur

Nanoporenherstellung in Graphen kann vorteilhafterweise ein thermisches Lithografieverfahren mittels einer Rastersonde, auch thermal scanning probe lithography oder kurz t-SPL genannt, in Kombination mit selbstorganisierenden Monoschichten eingesetzt werden, um darin bereits vor der Umwandlung der selbstorganisierenden Monoschichten zu Graphen mittels t-SPL Nanoporen zu definieren und somit zu erstellen, die nach der thermischen Umwandlung der selbstorganisierenden Monoschichten in der erzeugten Graphenschicht für die DNA-Sequenzierung oder DNA-Fragmentlängenbestimmung geeignete Nanoporen hinterlassen.

Solche Nanoporen können beispielsweise direkt in Graphen mittels einer AFM-Methode (AFM = atomic force microscope; "Rasterkraftmikroskop") mittels elektrischer Strompulse eingebrannt werden. Eine geeignete Ausstattung und geeignete Prozesse sind dafür jedoch nicht standardmäßig verfügbar oder können nicht kommerziell bezogen werden, sodass eine Adaption kommerziell erhältlicher AFM-Werkzeuge für diesen Zweck und eine entsprechende aufwendige Prozessentwicklung hinsichtlich der Methode des Nanolochbrennens in Graphen mittels elektrischer Stromimpulse erforderlich wären. Demgegenüber sind das t-SPL-Verfahren und entsprechende Prozesse zur Einbringung von Nanostrukturen in Polymere inzwischen kommerziell und kostengünstig verfügbar, und zwar sowohl in Form geeigneter Werkzeuge als auch in Form geeigneter Prozesse zur Nanostrukturierung polymerer Materialien. Graphen ist jedoch kein polymeres organisches Material, sodass die erwähnten Prozesse nicht ohne Weiteres auf Graphen übertragbar sind. Werden jedoch Nanoporen mittels t-SPL in der organischen Vorläuferschicht des Graphens in Form einer SAM-Beschichtung erzeugt, so ist die Technologie der thermischen Erzeugung von Nanoporen eins zu eins in die primäre Strukturierung der SAM-Beschichtung, also eines organischen Materials, übertragbar. Anschließend kann die derart strukturierte SAM-Beschichtung beispielsweise unter einer temporär aufgebrachten Metallschicht, etwa aus Kupfer oder Nickel, thermisch zu Graphen konvertiert werden, wodurch entsprechend der vorangehenden Strukturierung Nanoporen in der Graphenschicht erzeugt werden. Die Auflösungsgrenze der t-SPL-Methode liegt beispielsweise bei 10 bis 15 nm, was für den Verwendungszweck an sich zu groß ist, jedoch bei entsprechender Steuerung der thermischen Konversion der SAM-Beschichtung zu Graphen letztlich zu Nanoporen mit Durchmessern von wenigen Nanometern führen kann, wie sie für die Nanoporensequenzierung bzw. Nanoporen-DNA-Fragmentlängenbestimmung optimal sind. Damit ist eine kommerziell erhältliche und kostengünstige Technologie unmittelbar einsetzbar zur Nanostrukturierung von Polymeren (dem SAM-Beschichtungsmaterial), um über die Strukturierung dieses Ausgangsmaterials nachfolgend definierte Nanoporen in Graphen zu erzeugen.

Es wird ein Verfahren zum Herstellen einer Sequenziereinheit zum Sequenzieren eines biochemischen Materials vorgestellt, wobei das Verfahren folgende Schritte umfasst:
Erstellen zumindest einer Sequenzierpore zum Sequenzieren des biochemischen Materials in einer Vorläuferschicht in einem thermischen Lithografieverfahren, um eine vorstrukturierte Schicht zu erzeugen; und
Umwandeln der vorstrukturierten Schicht in eine Graphenschicht durch Erwärmen der vorstrukturierten Schicht auf eine Umwandlungstemperatur, um die Sequenziereinheit herzustellen.

Im _Schritt des Umwandelns kann die Sequenzierpore abhängig von der Umwandlungstemperatur auf ein zum Sequenzieren des biochemischen Materials geeignetes Maß verkleinert werden. Das geeignete Maß ergibt sich dabei beispielsweise aus einer Abmessung des biochemischen Materials. Für unterschiedliches biochemisches Material können unterschiedliche Durchmesser der Sequenzierporen erforderlich sein. Durch eine geeignete Auswahl der Umwandlungstemperatur können entsprechende Durchmesser erzeugt werden. Unter einem biochemischen Material kann beispielsweise eine Abfolge von Nukleotiden, etwa DNA, oder Ähnliches verstanden werden. Unter Sequenzierung kann die Bestimmung einer Abfolge von Bausteinen des biochemischen Materials verstanden werden, etwa einer Nukleotid-Abfolge in einem DNA-Molekül. Unter einer Sequenzierpore kann eine Öffnung verstanden werden, durch die das biochemische Material bei der Sequenzierung hindurchtreten kann. Unter einer Vorläuferschicht kann eine aus sogenannten Präkursormolekülen zusammengesetzte Schicht zur Herstellung einer Graphenschicht verstanden werden. Beispielsweise kann es sich bei der Vorläuferschicht um eine selbstorganisierende Monoschicht handeln.

Bei solchen Präkursoren kann es sich beispielsweise um organische Trichlorsilane, Trimethoxysilane oder Triethoxysilane, beispielsweise Octyltrichlorsilan, Decyltrichlorsilan, Octadecyltrichlorsilan, Phenyltrichlorsilan, Octyltrimethoxysilan, Decyltrimethoxysilan, Octadecyltrimethoxysilan, Phenyltrimethoxysilan, Octyltriethoxysilan, Decyltriethoxysilan, Octacecyltriethoxysilan oder Phenyltriethoxysilan, handeln. Besonders geeignet sind Trichlorsilane, da diese aufgrund ihrer hohen chemischen Reaktivität sehr schnell hydrolisieren und an Hydroxylgruppen hydrophiler Oberflächen binden und auch schnell quervernetzen. Es können jedoch auch Methoxy- oder Ethoxysilane eingesetzt werden. Für die Graphenerzeugung besonders geeignet sind Phenylsilane, insbesondere Trichlorphenylsilan, da diese Präkursormoleküle bereits aromatische Kohlenstoffringe enthalten, die der späteren Graphenstruktur entsprechen. Das Ausgangsmaterial ist im Fall von Phenyltrichlorsilan von seiner Struktur bereits ähnlich dem Zielmaterial, was die nachfolgende thermische Umwandlung der aromatischen Kohlenstoffringe zum Graphen fördert.

Unter einem thermischen Lithografieverfahren kann ein thermisch gesteuertes Verfahren der Nanolithografie verstanden werden, etwa unter Verwendung einer entsprechenden Rastersonde oder Tunnelspitze mit hoher Spitzentemperatur zum Einbrennen der Sequenzierpore über die hohe Temperatur der Spitze (t-SPL-Verfahren). Alternativ kann auch mittels eines elektrischen Tunnelstrompulses eine Nanopore in ein leitfähiges Ausgangsmaterial gebrannt werden.

Das hier vorgestellte Verfahren kann beispielsweise im Kontext eines Gesamtprozesses zur Herstellung von Arrayzellen in Silizium mit integrierten Nanoporen in Graphen durchgeführt werden. Dabei kann die Sequenziereinheit beispielsweise in einem Chip mit einem qPCR-Array kombiniert werden.

Ein Durchmesser der Sequenzierpore kann im Schritt des Umwandelns auf einen vorbestimmten Durchmesser verkleinert werden. Die Umwandlungstemperatur kann abhängig von dem vorbestimmten Durchmesser eingestellt werden. Der vorbestimmte Durchmesser kann abhängig von einer Charakteristik, beispielsweise Größe des zu sequenzierenden biochemischen Materials ausgewählt werden. Um unterschiedliche vorbestimmte Durchmesser zu erreichen können unterschiedliche Umwandlungstemperaturen eingesetzt und beispielsweise unter Verwendung einer Nachschlagetabelle ausgewählt werden.

Gemäß einer Ausführungsform kann im Schritt des Erstellens die Sequenzierpore in Form eines Nanopunktes definiert werden. Dadurch kann die Sequenzierpore besonders schnell erstellt werden, beispielsweise mittels einer temperierten Rastersonde.

Im Schritt des Erstellens kann die Sequenzierpore mit einem Durchmesser von weniger als 20 Nanometern, beispielsweise mit einem Durchmesser zwischen 10 und 20 Nanometern erstellt werden. Der Wertebereich von 10 bis 20 Nanometern ergibt sich dabei aus der momentanen Auflösungsgrenze des t-SPL Verfahrens, wobei kleinere Nanoporen gemäß einer Ausführungsform durch die darauffolgende "Zuwachsmethode" erreicht werden. Entsprechend kann im Schritt des Umwandelns die Sequenzierpore auf einen Durchmesser zwischen 1 und 5 Nanometern, besonders bevorzugt zwischen 1 und 2 Nanometern verkleinert werden. Dies ist eine Möglichkeit zur Erzielung kleiner Poren, wenn es nicht auf sinnvolle Weise möglich ist, direkt Nanoporen von 1 bis 5 Nanometern oder noch besser von 1 bis 2 Nanometern herzustellen. Da t-SPL und auch die meisten anderen Verfahren das nicht direkt können, wird als vorteilhafte Abhilfemaßnahme das erwähnte Zuwachsen der Pore eingesetzt. Das "heilt" gewissermaßen die unzureichende Auflösungsgrenze der t-SPL Methode. Diese Ausführungsform ermöglicht eine besonders effiziente Herstellung der Sequenziereinheit mit den gewünschten Porendurchmessern von 1 bis 5 Nanometern bzw. besonders bevorzugt 1 bis 2 Nanometern mit wenigen Fertigungsschritten.

Zudem kann dadurch im Schritt des Umwandelns die Graphenschicht zumindest im Randbereich der Sequenzierpore als Monoschicht ausgebildet werden. Dadurch wird eine möglichst präzise Fragmentlängenbestimmung, beispielsweise von DNA-Fragmenten, beim Durchtritt der Fragmente durch die Sequenzierpore ermöglicht.

Gemäß einer weiteren Ausführungsform kann im Schritt des Erstellens die Sequenzierpore in einer selbstorganisierenden Monoschicht als der Vorläuferschicht erstellt werden. Unter einer selbstorganisierenden Monoschicht kann im Allgemeinen eine sich bei Vorhandensein bestimmter Reaktionsbedingungen spontan auf einer oberflächenaktiven oder organischen Substanz bildende Schicht mit hoher innerer Ordnung und definierter Dicke verstanden werden. Dadurch kann die Sequenzierpore mit hoher Wiederholgenauigkeit, hoher Reinheit und hoher Qualität erstellt werden.

Des Weiteren kann das Verfahren einen Schritt des Aufbringens einer Metallschicht auf die vorstrukturierte Schicht umfassen. Dabei kann im Schritt des Umwandelns die vorstrukturierte Schicht mittels der Metallschicht umgewandelt werden. Bei der Metallschicht kann es sich beispielsweise um eine Schicht aus Kupfer oder Nickel oder einem sonstigen geeigneten Metall oder einer sonstigen geeigneten Metalllegierung handeln. Die Metallschicht kann unter anderem zur Erwärmung der vorstrukturierten Schicht dienen. Insbesondere kann es sich bei der Metallschicht um eine temporäre Schicht handeln, die nach dem Umwandeln wieder entfernt werden kann. Dadurch wird eine kontrollierte Erwärmung der vorstrukturierten Schicht und somit eine präzise Verkleinerung der Sequenzierpore mit hoher Wiederholgenauigkeit ermöglicht.

Es ist vorteilhaft, wenn im Schritt des Erstellens ein Porenabschnitt der Vorläuferschicht auf eine Temperatur oberhalb einer Zersetzungstemperatur der Vorläuferschicht erwärmt wird, um die Sequenzierpore im Porenabschnitt zu Erstellen. Unter einer Zersetzungstemperatur kann eine Temperatur verstanden werden, bei der sich die Vorläuferschicht in sauerstoffhaltiger Atmosphäre zersetzt. Durch diese Ausführungsform wird erreicht, dass die Vorläuferschicht im Porenabschnitt augenblicklich erodiert und somit die Sequenzierpore äußerst schnell in die Vorläuferschicht gleichsam eingebrannt wird.

Das Verfahren kann gemäß einer weiteren Ausführungsform einen Schritt des Erzeugens eines Kavernenelements mit zumindest einer eine Eintrittsöffnung und eine Austrittsöffnung aufweisenden Kaverne zum Aufnehmen des biochemischen Materials umfassen. Dabei kann die Vorläuferschicht vor dem Schritt des Erstellens auf einen die Austrittsöffnung aufweisenden Abschnitt des Kavernenelements aufgebracht werden und im Schritt des Erstellens die Sequenzierpore gegenüber der Austrittsöffnung erstellt werden. Unter einer Kaverne kann beispielsweise eine kanalartige Durchgangsöffnung im Kavernenelement verstanden werden. Das Kavernenelement kann auch mit mehreren Kavernen erzeugt werden. Beispielsweise kann das Kavernenelement als Kavernenwafer erzeugt werden. Die Kaverne kann beispielsweise als qPCR-Arrayzelle fungieren. Unter einer Eintrittsöffnung kann eine Öffnung verstanden werden, durch die die Kaverne mit dem biochemischen Material befüllt werden kann. Analog dazu kann unter einer Austrittsöffnung eine Öffnung verstanden werden, durch die das biochemische Material aus der Kaverne austreten kann. Durch diese Ausführungsform kann ein Querschnitt der Austrittsöffnung kontrolliert und genau auf ein zum Sequenzieren des biochemischen Materials geeigneten Durchmesser verringert werden.

Vorteilhaft ist es, wenn im Schritt des Erzeugens das Innere der Kaverne mit einer hydrophilen Beschichtung erzeugt wird. Zusätzlich oder alternativ kann ein die Umgebung der Eintrittsöffnung umfassender Abschnitt des Kavernenelements mit einer hydrophoben Beschichtung erzeugt werden. Durch die hydrophilen Eigenschaften des Kaverneninneren kann das Befüllen der Kaverne mit dem biochemischen Material vereinfacht werden. Zudem kann durch die Verbindung zwischen hydrophilem Kaverneninneren und der hydrophobem Umgebung der Kaverneneintrittsöffnung ein unkontrolliertes Auslaufen des biochemischen Materials, also eine sogenannte "Verschleppung" aus der Kaverne heraus vermieden werden.

Somit kann das Innere jeder Kaverne hydrophile Oberflächeneigenschaften aufweisen. Die Umgebung der Eintrittsöffnung auf der Oberfläche kann hingegen möglichst hydrophob beschichtet sein. Diese hydrophilen bzw. hydrophoben Schichten können wichtige Rollen ausüben. Gemäß einer Ausführungsform kann auf der Oberfläche des Kavernenelements, das heißt auf der Waferoberfläche bzw. Chipoberfläche, also um die Eintrittsöffnungen bzw. zwischen den Eintrittsöffnungen von Kavernen, eine hydrophobe Schichtwirkung erreicht werden. Damit kann vermieden werden, dass über die Oberfläche eine Verschleppung von Kaverneninhalten zu Nachbarkavernen erfolgt oder elektrische Strompfade zwischen Nachbarkavernen entstehen. Somit kann ein sogenannter "Cross-talk" vermieden werden, d.h. dass Bio-Material aus einer Kaverne über die Chipoberfläche in die Nachbarkaverne kriecht und umgekehrt.

Ein solcher Cross-talk ist unerwünscht, und zu dessen Vermeidung dient das relativ hydrophobe Si-Nitrid auf der Chipoberfläche. Die hydrophobe Wirkung der Oberfläche kann nach bekanntem Stand der Technik auch durch Bedrucken mit weiteren hydrophoben Schichten, z.B. als Self-assembled monolayer coating (z.B. mit PFOTS=Perfluorooctyltrichlorsilan, PFD(ecyl)TS, PFO(cta)D(ecyl)TS o.ä.) verstärkt werden. Das Innere der Kavernen selbst ist gemäß einer Ausführungsform möglichst hydrophil, um die Befüllung mit wässrigen Medien zu erleichtern und die Flüssigkeit im Kaverneninneren festzuhalten.

Der hier beschriebene Ansatz schafft ferner eine Sequenziereinheit zum Sequenzieren eines biochemischen Materials. Die Sequenziereinheit umfasst eine in einem Verfahren gemäß einer der vorstehenden Ausführungsformen hergestellte Graphenschicht mit zumindest einer Sequenzierpore zum Sequenzieren des biochemischen Materials.

Ausführungsbeispiele der Erfindung sind in den Zeichnungen dargestellt und in der nachfolgenden Beschreibung näher erläutert. Es zeigt:
- Fig. 1: eine schematische Darstellung einer Sequenziereinheit gemäß einem Ausführungsbeispiel;
- Fig. 2a-2h: eine schematische Darstellung eines Gesamtprozesses zur Herstellung einer Sequenziereinheit aus Fig. 1; und
- Fig. 3: ein Ablaufdiagramm eines Verfahrens gemäß einem Ausführungsbeispiel.

In der nachfolgenden Beschreibung günstiger Ausführungsbeispiele der vorliegenden Erfindung werden für die in den verschiedenen Figuren dargestellten und ähnlich wirkenden Elemente gleiche oder ähnliche Bezugszeichen verwendet, wobei auf eine wiederholte Beschreibung dieser Elemente verzichtet wird.

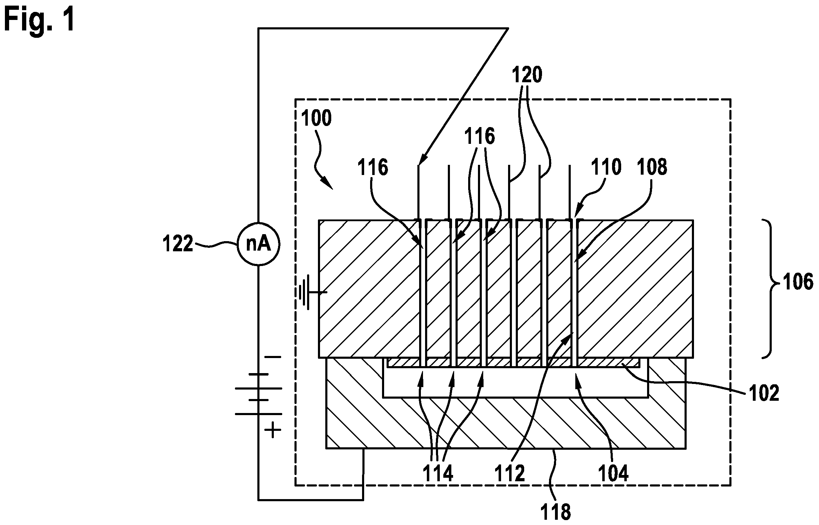

Fig. 1 zeigt eine schematische Darstellung einer Sequenziereinheit 100 gemäß einem Ausführungsbeispiel. Die Sequenziereinheit 100 umfasst eine Graphenschicht 102 mit einer Sequenzierpore 104 zum Sequenzieren eines biochemischen Materials, hier beispielhaft zur DNA-Fragmentlängenbestimmung.

Bei der Graphenschicht 102 handelt es sich um eine Schicht, die durch thermisches Umwandeln einer mit der Sequenzierpore 104 in einem thermischen Lithografieverfahren vorstrukturierten selbstorganisierenden Monoschicht erzeugt wurde. Eine Umwandlungstemperatur beim thermischen Umwandeln wurde so gewählt, dass die Sequenzierpore 104 durch Zuwachsen auf einen zum Sequenzieren des biochemischen Materials geeigneten Durchmesser verkleinert wurde. Je nach gewünschten Durchmesser der Sequenzierpore 104 kann somit eine geeignete Umwandlungstemperatur ausgewählt werden.

Gemäß diesem Ausführungsbeispiel bildet die Graphenschicht 102 einen Boden eines Kavernenelements 106 mit einer Kaverne 108 zum Vorlagern des biochemischen Materials. Die Kaverne 108 ist beispielhaft als ein das Kavernenelement 106 im Wesentlichen geradlinig durchquerender Kanal mit einer Eintrittsöffnung 110 und einer der Eintrittsöffnung gegenüberliegenden Austrittsöffnung 112 ausgeformt. Die Sequenzierpore 104 liegt der Austrittsöffnung 112 gegenüber, sodass ein Austrittsquerschnitt der Kaverne 108 durch die Sequenzierpore 104 definiert ist.

Gemäß dem in Fig. 1 gezeigten Ausführungsbeispiel weist die Graphenschicht 102 eine Mehrzahl weiterer Sequenzierporen 114 zum Sequenzieren des biochemischen Materials oder weiterer biochemischer Materialien auf. Die weiteren Sequenzierporen 114 sind analog zur Sequenzierpore 104 erzeugt. Das Kavernenelement 106 ist dementsprechend mit mehreren weiteren Kavernen 116 zum Aufnehmen der biochemischen Materialien ausgeformt, wobei den jeweiligen Austrittsöffnungen der weiteren Kavernen 116 je eine weitere Sequenzierpore 114 gegenüberliegt.

Die Sequenziereinheit 100 ist beispielsweise in eine Lab-on-Chip-Umgebung integrierbar, angedeutet durch einen gestrichelten Rahmen. Beispielhaft ist die Sequenziereinheit 100 gemäß Fig. 1 als gebondetes Bauteil aus einem Kavernenwafer mit Nanoporen am Boden und einer kappenförmigen Gegenelektrode 118 realisiert. Die Graphenschicht 102 ist durch die Gegenelektrode 118 abgedeckt. Die Sequenziereinheit 100 ist über die Gegenelektrode 118 und mehrere weitere Elektroden 120, die je an einer Eintrittsöffnung der Kavernen 108, 116 angeordnet sind, mit einer Messeinrichtung 122 zur Strommessung angeschlossen. Insbesondere ist die Sequenziereinheit 100 als LATE-qPCR-Sanger-Sequenzierarray in Silizium mit nachgeschaltetem graphenbasierten Nanoporen- Sequencer über DNA-Fragmentlängenbestimmung realisiert.

Als Vorläuferschicht zum Erstellen der Sequenzierporen in dem thermischen Lithografieverfahren, insbesondere durch thermische Rastersondenlithografie, kurz t-SPL, bieten sich selbstorganisierende Monoschichten, nachfolgend kurz Monoschichten genannt, in geradezu idealer Weise an, da Präzision und Reproduzierbarkeit dieser Strukturierungsmethode aufgrund der sehr geringen und äußerst homogenen, da monomolekularen Schichtdicke besonders gut sind. Darüber hinaus erzeugt die t-SPL-Methode bei einer Temperatur der AFM-Spitze von 900 bis 950 °C auf der Schichtoberfläche lokal und instantan Temperaturen von 300 bis 400 °C, was deutlich oberhalb der Zersetzungstemperatur der Monoschicht in sauerstoffhaltiger Umgebung, beispielsweise in Luft oder in mit Sauerstoff angereicherter Atmosphäre oder in reinem Sauerstoff, liegt. Die Zersetzungstemperatur liegt beispielsweise bei ca. 250 °C. Dadurch setzt die Erosion der Monoschicht im betroffenen Bereich augenblicklich ein, sodass das "Brennen" einer Nanopore, vorangehend auch Sequenzierpore genannt, in die Monoschicht äußerst schnell erfolgen kann. Da nur vergleichsweise wenige Nanoporen über die Waferfläche erzeugt werden, beispielsweise maximal zwei Nanoporen pro qPCR-Arrayzelle, und dafür jeweils nur ein oder maximal zwei Punkte pro qPCR-Arrayzelle angefahren und als Punkte "gebrannt" werden, ist der Herstellungsprozess der Sequenziereinheit 100 auch sehr wirtschaftlich, da die Probleme, die im Zusammenhang mit einem dichten Beschreiben großer Flächen mit einer Vielzahl unterschiedlich dimensionierter und ausgedehnter Strukturen auftreten können, vermieden werden. Vielmehr genügt hier pro qPCR-Arrayzelle das Anfahren von jeweils einem oder maximal zwei Positionen und das "Brennen" von jeweils einem einzigen Nanopunkt oder Nanodot an jeder Position. Vorteilhafterweise werden also Punkte und keine komplexe Strukturgeometrien im Nanometer- und Mikrometerbereich geschrieben.

Die Auflösungsgrenze der t-SPL-Methode liegt typischerweise zwischen 10 und 15 nm. Für Nanoporen zur DNA-Sequenzierung über DNA-Fragmentlängenbestimmung wäre das etwas zu groß und würde die elektrischen Signale oder den elektrischen Signalhub deutlich reduzieren. Der Mechanismus der Konversion der Monoschicht in die Graphenschicht 102 verbessert die Situation signifikant, weil in der Monoschicht für die Graphenbildung üblicherweise ein Kohlenstoffüberschuss vorliegt, der zur Bildung von Bi- oder Trilayergraphen führt, also mehrerer Graphenlagen statt einer einzelnen Graphenlage, auch Monolayer genannt. Durch den Kohlenstoffüberschuss kommt es im Bereich der Nanoporen zu einem "Zuwachsen" ausgehend von den Rändern der Nanoporen, sodass die Nanoporen im Graphen deutlich kleiner ausfallen, als sie primär in der Monoschicht erzeugt wurden. Bei optimaler Prozessführung des thermischen Annealings kann somit eine Größe der Nanoporen von 1 bis 2 nm eingestellt werden, wobei sich der Charakter der Graphenschicht 102 mit zunehmender Annäherung an eine Nanopore von einem Bi- oder Trilayer zu einem Monolayer verändert.

Damit werden für die Applikation der Nanoporensequenzierung durch eine möglichst präzise DNA-Fragmentlängenbestimmung über die Strommodulation beim Fragmentdurchtritt durch die Nanopore ideale Verhältnisse erreicht: Die Membrandicke unmittelbar an der Porenöffnung entspricht etwa einer Graphenmonolage bei einem Porendurchmesser von 1 bis 2 nm.

Nachfolgend wird beispielhaft ein Gesamtprozess zur Integration der Graphenherstellung mit Nanoporenerzeugung durch Einsatz selbstorganisierender Monoschichten und t-SPL in die Herstellung eines Sequenzier-qPCR-Arrays näher beschrieben. Die angegebenen Dimensionen, Schichtdicken oder verwendeten Schichtmaterialien im Prozessfluss sind lediglich als beispielhafte Angaben zu verstehen.

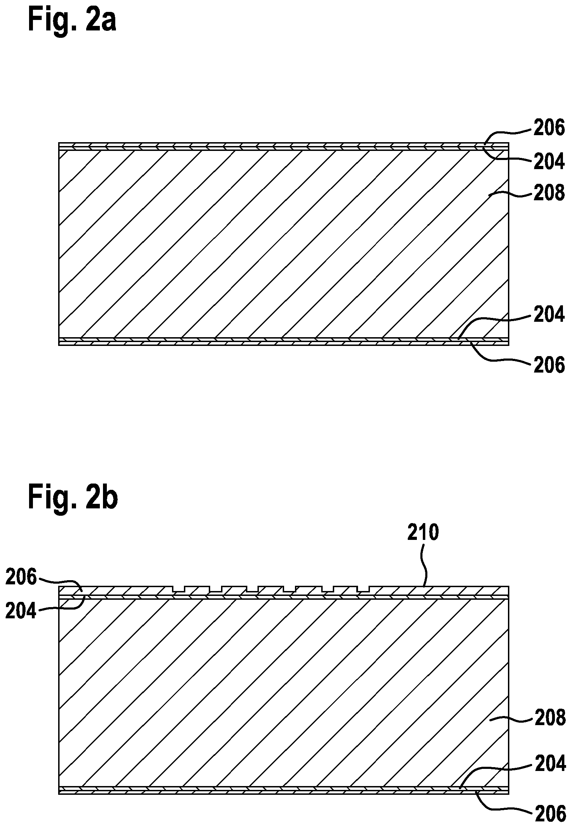

Die Figuren 2a bis 2h zeigen schematisch einen Gesamtprozess zur Herstellung einer Sequenziereinheit aus Fig. 1. Zu Prozessbeginn wird eine Schichtfolge von beispielsweise 250 nm Siliziumdioxid 204 durch thermische Oxidation und darüber 280 nm Siliziumnitrid 206 durch Niederdruckgasphasenabscheidung ganzflächig auf eine Vorder- und Rückseite eines Siliziumwafers 208 aufgewachsen. Dies ist in Fig. 2a gezeigt. Die Druckspannung des thermischen Siliziumdioxids wird kompensiert bzw. überkompensiert durch die Zugspannung des abgeschiedenen Siliziumnitrids. Die angegebenen Schichtdicken sollen nur illustrieren, wie durch das Zusammenspiel von druck- und zugverspannten Schichten durch entsprechende Dimensionierung der Schichtdicken insgesamt eine Zugspannung im Schichtaufbau erzeugt werden kann. Die resultierende Zugspannung ist im weiteren Verlauf des Prozesses wichtig, um ein Brechen von freigestellten Schichtmembranen durch "Buckeln" zu vermeiden und eine ausreichende Stabilität sicherzustellen.

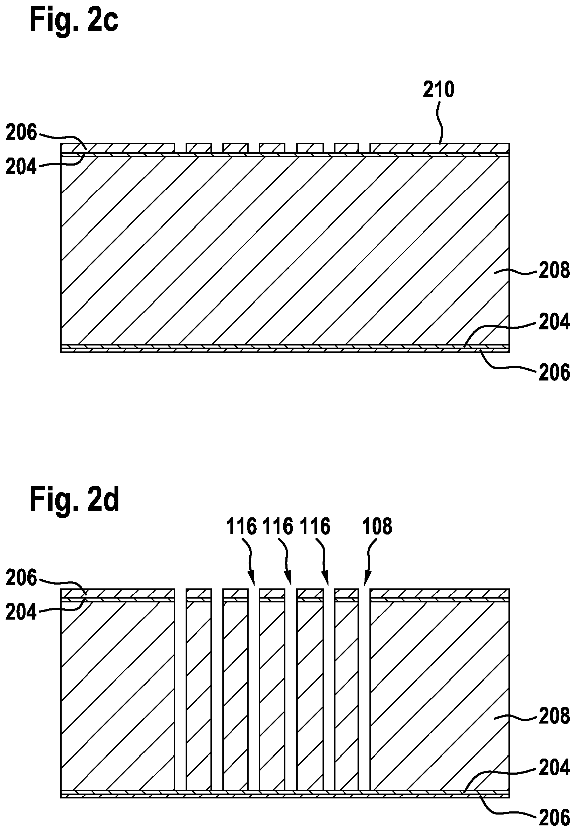

In einem weiteren Prozessschritt werden durch Fotolithografie mit einer hinreichend dicken Fotolackmaske 210 auf der Wafervorderseite die Geometrien von qPCR-Arrayzellelementen definiert, wie in Fig. 2b gezeigt. In einem selbstjustierten Prozess werden zunächst die Maskenfenster in der Siliziumnitridschicht 206 und der Siliziumdioxidschicht 204 durch Ätzen von der Wafervorderseite geöffnet. Dies ist in Fig. 2c gezeigt. Danach werden mit derselben Maskierung und mittels eines DRIE-Prozesses (DRIE = deep reactive ion etching; "reaktives lonentiefenätzen") die Kavernen 108, 116 durch den Siliziumwafer 208 von der Wafervorderseite bis zum Stopp auf dem Siliziumdioxid 204 der Waferrückseite geätzt. Das Ergebnis ist in Fig. 2d gezeigt. Die Kavernen 108, 116 stellen die späteren qPCR-Arrayzellelemente dar. Die Dicke der Siliziumdioxidschicht 204 toleriert eine gewisse Überätzung zum Ausgleich von Ätzraten-Uniformitätsabweichungen über die Waferfläche. Beispielhaft wird angenommen, dass bei diesem Überätzen bis zu 100 nm Siliziumdioxid im Kavernenbereich verloren gehen können. Die Siliziumdioxidschicht 204 weist also gemäß der beispielhaft gewählten Anfangsschichtdicke von 250 nm noch eine Restdicke von 150 nm auf. Die Siliziumdioxidschicht 204 sollte während des Überätzens nicht bis zur Siliziumnitridschicht 206 durchbrechen, weil Siliziumnitrid keine ausreichende Stabilität im DRIE-Prozess aufweist. Für den Fachmann ist offensichtlich, durch welche Maßnahmen hinsichtlich der Oxiddicke eine Anpassung an eine höhere oder geringere zu tolerierende Überätzung erfolgen kann. Die Membranen der Kavernenböden sind nach dem Durchätzen des Siliziums jeweils freitragend, sollten also wie vorstehend erwähnt zugverspannt sein, um nicht zu brechen und eine hinreichende Stabilität für die Folgeprozesse aufzuweisen.

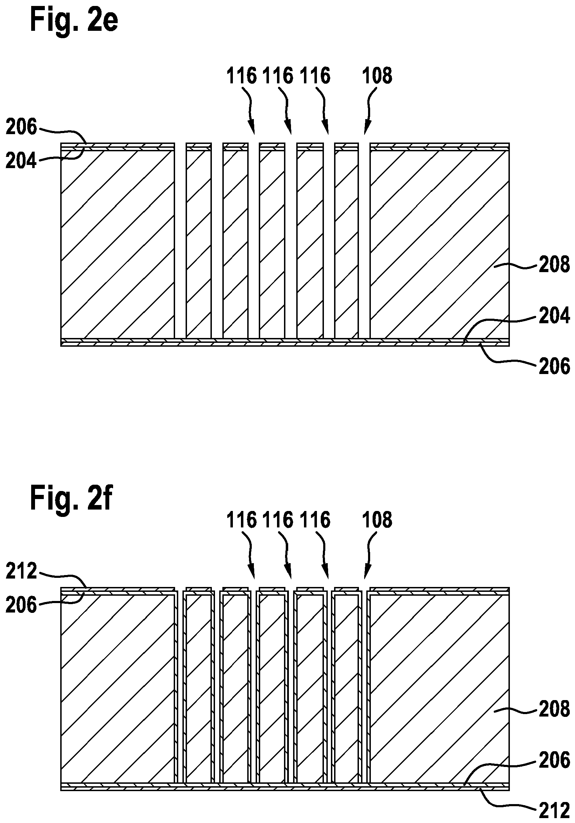

Anschließend wird, wie in Fig. 2e gezeigt, die Fotolackmaske 210 von der Wafervorderseite entfernt und der Siliziumwafer 208 thermisch oxidiert, wobei in den qPCR-Arrayzellelementen auf den Seitenwänden der Kavernen 108, 116 beispielsweise 2,5 µm Siliziumdioxid aufgewachsen wird. Infolge dieses thermischen Oxidationsprozesses wird ein Teil der Siliziumnitridschicht 206 ebenfalls oxidiert, allerdings sehr viel langsamer, als das auf freien Siliziumflächen der Fall ist. Für 2,5 µm thermisch auf Silizium gewachsenes Siliziumdioxid kann typischerweise von 30 nm Siliziumnitrid ausgegangen werden, das in ein ca. 40 nm dickes Re-Oxid 212, also Siliziumdioxid, umgewandelt wird, wie aus Fig. 2f ersichtlich.

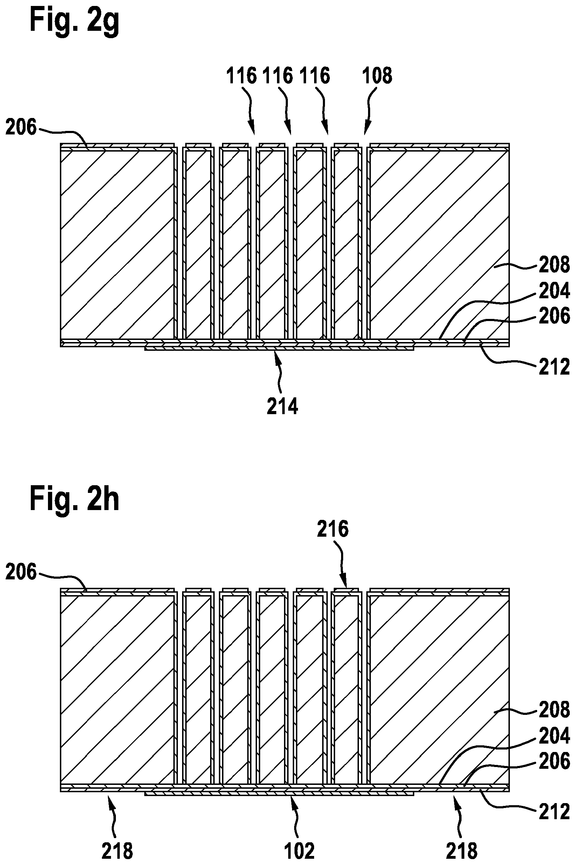

Das Re-Oxid 212 besitzt hydrophile Oberflächeneigenschaften. Wird es vorderseitig im Folgeprozess durch selektives Ätzen, beispielsweise mit gepufferter Flusssäure, von der Wafervorderseite wieder entfernt, so entsteht eine vergleichsweise hydrophobe Wafervorderseite von Siliziumnitrid, wie in Fig. 2g gezeigt. Bei diesem Ätzvorgang gehen in den Kavernen 108, 116 an den Seitenwänden und am Kavernenboden ebenfalls ca. 50 bis 100 nm Siliziumdioxid verloren, sodass am Kavernenboden minimal 50 nm Siliziumdioxid 204 mit dem darüberliegenden Schichtaufbau aus ca. 250 nm Siliziumnitrid und 40 nm Re-Oxid 212 verbleiben, wobei das Re-Oxid 212 von der Waferrückseite nicht entfernt werden sollte. Da die Waferrückseite dank des Re-Oxids 212 hydrophil bleibt, kann nun der Beschichtungsprozess zum Beschichten der Waferrückseite mit einer Vorläuferschicht 214, hier einer selbstorganisierenden Monoschicht, zum Erzeugen der Graphenschicht durchgeführt werden. Die Monoschicht 214 wird nach der Abscheidung mittels t-SPL mit Nanoporen versehen. Anschließend wird eine Metallschicht auf die Waferrückseite aufgedampft, beispielsweise 300 nm Kupfer oder Nickel. Die darunter liegende Monoschicht 214 wird thermisch zu Graphen umgewandelt. Der Temperprozess bestimmt dabei auch die Größe der Nanoporen, da während der Temperaturbehandlung der Kohlenstoff eine gewisse Mobilität aufweist und die 10 bis 15 nm großen Poren ausgehend von der t-SPL-Strukturierung der Monoschicht 214 von den Rändern her langsam zuwachsen.

Nach Entfernung der Metallschicht liegt die Graphenschicht 102 mit den erzeugten Nanoporen frei auf dem Schichtaufbau des Kavernenbodens. Dies ist in Fig. 2h gezeigt. Nun wird der Schichtaufbau des Kavernenbodens schrittweise bis zu den Kavernen 108, 114 durchgeätzt, und zwar vorzugsweise mittels isotropisch ätzender selektiver Nassätzchemien, wobei die Graphenschicht 102 als Maskierung für den Ätzprozess dient. Ausgehend von den Nanoporen werden die Siliziumdioxidschichten nacheinander beispielsweise mittels stark verdünnter oder gepufferter Flusssäure von der Waferrückseite her durchgeätzt, während für das Durchätzen der Siliziumnitridzwischenschicht beispielsweise Phosphorsäure verwendet wird. Für den selbstjustierten Ätzvorgang mithilfe der mit Nanoporen versehenen Graphenschicht 102 als Maskierung wird also in drei Schritten verdünnte (gepufferte) Flusssäure für das Re-Oxid 212, Phosphorsäure für das Siliziumnitrid 206 und verdünnte (gepufferte) Flusssäure für das darunter liegende Siliziumdioxid 204 verwendet. Als besonderer Vorteil wird bei diesem Vorgehen mit Benutzung der Graphennanoporen als Maskierschicht für die Ätzung der darunterliegenden Dielektrika erreicht, dass diese Dielektrika außerhalb der Nanoporen im Wesentlichen erhalten bleiben, die Graphenschicht 102 mechanisch stützen und somit für zusätzliche Stabilität sorgen.

Nach Erzeugung einer Metallisierung 216 auf der Wafervorderseite und der Bondung eines Gegenwafers auf Anschlussflächen 218 an der Waferrückseite liegt ein Array-Zellenaufbau zum Anschluss an eine Messeinrichtung vor, wie er in Fig. 1 schematisch dargestellt ist.

Ein weiterer Vorteil dieses Prozessablaufs liegt darin, dass die Wafervorderseite dank der Siliziumnitridschicht 206 relativ hydrophob ist, während die Kavernenwände in den qPCR-Arrayzellen dank der Siliziumdioxidschicht 204 auf den Seitenwänden und am Kavernenboden hydrophil sind. Damit gelingt es in einfacher Weise, die qPCR-Arrayzellen mit wässrigen Medien zu befüllen, da diese in die Arrayzellen durch die hydrophile Umgebung geradezu eingesaugt und darin festgehalten werden. Darüber hinaus kann der vorderseitige Verschluss der Arrayzellen mittels Ölen, vorzugsweise mittels dielektrisch hochfester und hochisolierender Fluoröle oder Fluorcarbone wie Perfluorpolyether oder FC40 oder FC77 (Hersteller: 3M), besonders gut, zuverlässig und reproduzierbar erfolgen, da die hydrophobe Wafervorderseite einen Wasserfilm abweist bzw. ein Wasserfilm von den nachrückenden Fluorölen oder Fluorcarbonen von dort sehr leicht und vollständig verdrängt werden kann. Dadurch wird sowohl ein elektrisches als auch ein biochemisches Übersprechen zwischen den Arrayzellelementen wirksam unterdrückt, da ein die Arrayzellelemente verbindender Wasserfilm durch die hydrophoben Oberflächeneigenschaften wirksam unterdrückt wird. Die hydrophobe Wirkung der Oberfläche kann bei Bedarf noch weiter verstärkt werden, beispielsweise durch Bedrucken mit Perfluoralkyl-trichlorsilanen wie z.B. Perfluorooctyltrichlorsilan oder Perfluorodecyltrichlorsilan.

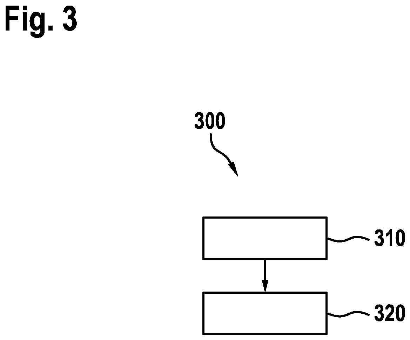

Fig. 3 zeigt ein Ablaufdiagramm eines Verfahrens 300 gemäß einem Ausführungsbeispiel. Das Verfahren 300 kann beispielsweise zum Herstellen einer vorangehend anhand der Figuren 1 bis 2h beschriebenen Sequenziereinheit durchgeführt werden. Dabei wird in einem Schritt 310 in einem thermischen Lithografieverfahren durch Erstellen zumindest einer Sequenzierpore in einer Vorläuferschicht, insbesondere einer selbstorganisierenden Monoschicht, eine vorstrukturierte Schicht erzeugt. Diese wird in einem weiteren Schritt 320 durch Erwärmen auf eine bestimmte Umwandlungstemperatur in eine Graphenschicht umgewandelt. Dabei wird die Sequenzierpore abhängig von der gewählten Umwandlungstemperatur um ein bestimmtes Maß verkleinert.

Mittels eines solchen Verfahrens können beispielsweise Nanoporen von größer 10 nm besonders wirtschaftlich mittels kommerziell erhältlicher Anlagen und kommerziell verfügbarer Prozesse in eine selbstorganisierende Monoschicht geschrieben werden und in einem nachfolgenden thermischen Prozess unter einer temporär aufgebrachten Metallschicht zu einer Graphenschicht mit entsprechenden Nanoporen umgewandelt werden. Dank Kohlenstoffmigration bei hohen Temperaturen können die Nanoporen im Graphen deutlich unter die Auflösungsgrenze von 10 bis 15 nm des thermischen Lithografieprozesses verkleinert werden. Darüber hinaus wird vorteilhafterweise eine Schichtdickenreduktion der erzeugten Graphenschicht unmittelbar um die Nanoporen herum erreicht, typischerweise von einer Bi- oder Trilayer-Struktur hin zu einer Monolayer-Struktur, was die Sequenziergenauigkeit bzw. die Genauigkeit der DNA-Fragmentlängenbestimmung erhöht. Da nur vergleichsweise wenige Nanoporen als Punkte auf den Wafern geschrieben werden, beispielsweise maximal zwei Nanoporen pro qPCR-Arrayzelle, ist der Prozess der Nanoporenerzeugung mittels t-SPL äußerst wirtschaftlich und schnell durchführbar.

Umfasst ein Ausführungsbeispiel eine "und/oder"-Verknüpfung zwischen einem ersten Merkmal und einem zweiten Merkmal, so ist dies so zu lesen, dass das Ausführungsbeispiel gemäß einer Ausführungsform sowohl das erste Merkmal als auch das zweite Merkmal und gemäß einer weiteren Ausführungsform entweder nur das erste Merkmal oder nur das zweite Merkmal aufweist.

## Patentansprüche

1. Verfahren (300) zum Herstellen einer Sequenziereinheit (100) zum Sequenzieren eines biochemischen Materials, wobei das Verfahren (300) folgende Schritte umfasst:
Erstellen (310) zumindest einer Sequenzierpore (104, 114) zum Sequenzieren des biochemischen Materials in einer Vorläuferschicht (214) in einem thermischen Lithografieverfahren, um eine vorstrukturierte Schicht zu erzeugen; und
Umwandeln (320) der vorstrukturierten Schicht in eine Graphenschicht (102) durch Erwärmen der vorstrukturierten Schicht auf eine Umwandlungstemperatur, um die Sequenziereinheit (100) herzustellen.

2. Verfahren (300) gemäß Anspruch 1, bei dem ein Durchmesser der Sequenzierpore (104, 114) im Schritt des Umwandelns (320) auf einen vorbestimmten Durchmesser verkleinert wird und die Umwandlungstemperatur abhängig von dem vorbestimmten Durchmesser eingestellt wird.

3. Verfahren (300) gemäß einem der vorangegangenen Ansprüche, bei dem im Schritt des Erstellens (310) die Sequenzierpore (104, 114) in Form eines Nanopunktes definiert wird.

4. Verfahren (300) gemäß einem der vorangegangenen Ansprüche, bei dem im Schritt des Erstellens (310) die Sequenzierpore (104, 114) mit einem Durchmesser zwischen 10 und 20 Nanometern erstellt wird.

5. Verfahren (300) gemäß einem der vorangegangenen Ansprüche, bei dem im Schritt des Umwandelns (320) die Sequenzierpore (104, 114) auf einen Durchmesser zwischen 1 und 5 Nanometern, insbesondere auf einen Durchmesser zwischen 1 und 2 Nanometern verkleinert wird.

6. Verfahren (300) gemäß einem der vorangegangenen Ansprüche, bei dem im Schritt des Umwandelns (320) die Graphenschicht (102) zumindest im Randbereich der Sequenzierpore (104, 114) als Monoschicht ausgebildet wird.

7. Verfahren (300) gemäß einem der vorangegangenen Ansprüche, bei dem im Schritt des Erstellens (310) die Sequenzierpore (104, 114) in einer selbstorganisierenden Monoschicht als der Vorläuferschicht (214) erstellt wird.

8. Verfahren (300) gemäß einem der vorangegangenen Ansprüche, mit einem Schritt des Aufbringens einer Metallschicht auf die vorstrukturierte Schicht, wobei im Schritt des Umwandelns (320) die vorstrukturierte Schicht mittels der Metallschicht umgewandelt wird.

9. Verfahren (300) gemäß einem der vorangegangenen Ansprüche, bei dem im Schritt des Erstellens (310) ein Porenabschnitt der Vorläuferschicht (214) auf eine Temperatur oberhalb einer Zersetzungstemperatur der Vorläuferschicht (214) erwärmt wird, um die Sequenzierpore (104, 114) im Porenabschnitt zu Erstellen.

10. Verfahren (300) gemäß einem der vorangegangenen Ansprüche, mit einem Schritt des Erzeugens eines Kavernenelements (106) mit zumindest einer eine Eintrittsöffnung (110) und eine Austrittsöffnung (112) aufweisenden Kaverne (108) zum Aufnehmen des biochemischen Materials, wobei die Vorläuferschicht (214) vor dem Schritt des Erstellens (310) auf einen die Austrittsöffnung (112) aufweisenden Abschnitt des Kavernenelements (106) aufgebracht wird und im Schritt des Erstellens (310) die Sequenzierpore (104) gegenüber der Austrittsöffnung (112) erstellt wird.

11. Verfahren (300) gemäß Anspruch 10, bei dem im Schritt des Erzeugens das Innere der Kaverne mit einer hydrophilen Beschichtung erzeugt wird und/oder ein die Eintrittsöffnung (110) umfassender Abschnitt des Kavernenelements (106) mit einer hydrophoben Beschichtung erzeugt wird.

## Claims

1. Method (300) for producing a sequencing unit (100) for sequencing a biochemical material, the method (300) comprising the following steps:
creating (310) at least one sequencing pore (104, 114) for sequencing the biochemical material in a precursor layer (214) in a thermal lithography method in order to generate a prestructured layer; and
converting (320) the prestructured layer into a graphene layer (102) by heating the prestructured layer to a conversion temperature in order to produce the sequencing unit (100).

2. Method (300) according to Claim 1, in which a diameter of the sequencing pore (104, 114) is reduced in size to a predetermined diameter in the conversion (320) step and the conversion temperature is set depending on the predetermined diameter.

3. Method (300) according to either of the preceding claims, in which the sequencing pore (104, 114) is defined in the form of a nanopoint in the creation (310) step.

4. Method (300) according to any of the preceding claims, in which the sequencing pore (104, 114) is created with a diameter between 10 and 20 nanometers in the creation (310) step.

5. Method (300) according to any of the preceding claims, in which the sequencing pore (104, 114) is reduced in size to a diameter between 1 and 5 nanometers, especially to a diameter between 1 and 2 nanometers, in the conversion (320) step.

6. Method (300) according to any of the preceding claims, in which the graphene layer (102) is formed as a monolayer at least in the edge region of the sequencing pore (104, 114) in the conversion (320) step.

7. Method (300) according to any of the preceding claims, in which the sequencing pore (104, 114) is created in a self-assembled monolayer as the precursor layer (214) in the creation (310) step.

8. Method (300) according to any of the preceding claims, comprising a step of applying a metal layer to the prestructured layer, the prestructured layer being converted by means of the metal layer in the conversion (320) step.

9. Method (300) according to any of the preceding claims, in which a pore segment of the precursor layer (214) is heated to a temperature above a decomposition temperature of the precursor layer (214) in the creation (310) step in order to create the sequencing pore (104, 114) in the pore segment.

10. Method (300) according to any of the preceding claims, comprising a step of generating a cavern element (106) comprising at least one cavern (108) having an entry opening (110) and an exit opening (112) for accommodating the biochemical material, the precursor layer (214) being applied before the creation (310) step to a segment of the cavern element (106) that has the exit opening (112), and the sequencing pore (104) being created opposite the exit opening (112) in the creation (310) step.

11. Method (300) according to Claim 10, in which, in the generation step, the interior of the cavern is generated with a hydrophilic coating and/or a segment of the cavern element (106) that comprises the entry opening (110) is generated with a hydrophobic coating.

## Revendications

1. Procédé (300) de fabrication d'une unité de séquençage (100) pour le séquençage d'un matériau biochimique, le procédé (300) comprenant les étapes suivantes :
création (310) d'au moins un pore de séquençage (104, 114) pour le séquençage du matériau biochimique dans une couche de précurseur (214) dans un procédé de lithographie thermique, pour produire une couche préstructurée ; et
conversion (320) de la couche préstructurée en une couche de graphène (102) par chauffage de la couche préstructurée à une température de conversion pour fabriquer l'unité de séquençage (100).

2. Procédé (300) selon la revendication 1, dans lequel un diamètre du pore de séquençage (104, 114) est, dans l'étape de conversion (320), réduit à un diamètre prédéfini, et la température de conversion est ajustée en fonction du diamètre prédéfini.

3. Procédé (300) selon l'une des revendications précédentes, dans lequel, dans l'étape de création (310), le pore de séquençage (104, 114) est défini sous forme d'un nanopoint.

4. Procédé (300) selon l'une des revendications précédentes, dans lequel, dans l'étape de création (310), le pore de séquençage (104, 114) est créé avec un diamètre entre 10 et 20 nanomètres.

5. Procédé (300) selon l'une des revendications précédentes, dans lequel, dans l'étape de conversion (320), le pore de séquençage (104, 114) est réduit à un diamètre entre 1 et 5 nanomètres, en particulier à un diamètre entre 1 et 2 nanomètres.

6. Procédé (300) selon l'une des revendications précédentes, dans lequel, dans l'étape de conversion (320), la couche de graphène est, au moins dans la zone marginale du pore de séquençage (104, 114), réalisée sous forme d'une monocouche.

7. Procédé (300) selon l'une des revendications précédentes, dans lequel, dans l'étape de création (310), le pore de séquençage (104, 114) est créé dans une monocouche autoorganisée servant de couche de précurseur (214).

8. Procédé (300) selon l'une des revendications précédentes, comprenant une étape d'application d'une couche métallique sur la couche préstructurée, la couche préstructurée étant, dans l'étape de conversion (320), convertie à l'aide de la couche métallique.

9. Procédé (300) selon l'une des revendications précédentes, dans lequel, dans l'étape de création (310), un segment de pore de la couche de précurseur (214) est chauffé à une température supérieure à une température de décomposition de la couche de précurseur (214), pour créer le pore de séquençage (104, 114) dans le segment de pore.

10. Procédé (300) selon l'une des revendications précédentes, comportant une étape production d'un élément caverne (106), comportant au moins une caverne (108) présentant un orifice d'entrée (110) et un orifice de sortie (112), pour recevoir le matériau biochimique, la couche de précurseur (214) étant, avant l'étape de création (310), appliquée sur un segment, comportant l'orifice de sortie (112), de l'élément caverne (106), et, dans l'étape de création (310), le pore de séquençage (104) étant créé en un point opposé à l'orifice de sortie (112).

11. Procédé (300) selon la revendication 10, dans lequel, dans l'étape de production, l'intérieur de la caverne est produit avec un revêtement hydrophile et/ou un segment, comprenant l'orifice d'entrée (110), de l'élément caverne (106) est produit avec un revêtement hydrophobe.
